# EUROPÄISCHE PATENTSCHRIFT

(11) **EP 0 574 608 B1**
(45) Veröffentlichungstag und Bekanntmachung des Hinweises auf die Patenterteilung: **19.08.1998**
(21) Anmeldenummer: 92110292.7
(22) Anmeldetag: 17.06.1992
(51) Int. Cl.: A61N 1/39

(54) **Defibrillator/Kardiovertierer**
Defibrillator/cardioverter
Defibrillateur/cardioverteur

(43) Veröffentlichungstag der Anmeldung: 22.12.1993
(73) Patentinhaber: Pacesetter AB, 171 95 Solna (SE)
(72) Erfinder: Hirschberg, Jakub, S-183 44 Täby (SE); Obel, Martin, S-182 33 Danderyd (SE)
(74) Vertreter: Lettström, Richard Wilhelm

(56) Entgegenhaltungen:
- EP-A- 0 315 368
- EP-A- 0 392 099
- EP-A- 0 480 569
- GB-A- 1 337 824
- GB-A- 2 085 593
- US-A- 3 895 639
- MEDICAL INSTRUMENTATION Bd. 20, Nr. 3, Mai 1986, Seiten 138 - 142 J.D. BOURLAND ET AL. 'sequential pulse defibrillation for implantable defibrillators'

## Beschreibung

Die Erfindung betrifft einen Defibrillator/Kardiovertierer, system mit einer Mehrzahl von n n≥3 im Bereich des Herzens anordnungsbaren Elektroden, die an einer Einrichtung zur Erzeugung von elektrischen Impulsen angeschlossen sind.

Bei einem derartigen, aus der DE-A-39 19 498 bekannten Defibrillator oder Kardiovertierer ist eine von mehreren Elektroden im Herzinneren angeordnet, und die übrigen Elektroden sind außen am Herzen plaziert. Die äußeren Elektroden sind elektrisch miteinander verbunden und an einemen ersten von zwei Ausgangsanschlüssen des Ausgangs eines Impulsgenerators angeschlossen; die im Herzinneren angeordnete Elektrode ist mit dem zweiten Ausgangsanschluß verbunden. Damit wird erreicht, daß sich bei der Abgabe eines elektrischen Impulses durch den Impulsgenerator die elektrische Stromdichte im Herzmuskel entsprechend der Anordnung der Elektroden verteilt und dabei vorzugsweise die dicksten Zonen des Herzmuskels, die den Haupteil der Herzmuskelmasse bilden, durchdringt, um eine Defibrillation bzw. Kardioversion zu erzielen. Die Stromverteilung läßt sich nur durch die Anordnung und Größe der einzelen Elektroden einstellen, wobei jedoch die Anordnung der Elektroden und insbesondere ihr Abstand zueinander durch die anatomischen Gegebenheiten beschränkt ist.

Aus der US-A-4 548 203 ist ein weiterer Defibrillator mit mehreren Elektroden bekannt, die paarweise an unterschiedlichen Ausgängen eines Impulsgenerators angeschlossen sind und an unterschiedlichen, vorzugsweise einander gegenüberliegenden Stellen des Herzens plaziert sind. Zur Defibrillation des Herzens werden die einzelnen Elektrodenpaare nacheinander über die Ausgänge des Impulsgenerators mit jeweils einem elektrischen Impuls beaufschlagt, wobei die Impulse jeweils durch ein Zeitintervall voneinander getrennt sind. Durch die sowohl räumlich als auch zeitlich getrennte Impulsabgabe soll eine Herabsetzung der zur Auslösung der Defibrillation erforderlichen Energie erreicht werden. Da jedoch jeweils nur zwei Elektroden gleichzeitig bei der Impulsabgabe beteiligt sind, ist eine echte Verteilung der Stromdichte auf unterschiedliche Zonen des Herzmuskels kaum möglich.

Aus der EP-B1-0 315 368 ist eine weitere Defibrillator-Einrichtung bekannt, die mit einem Kondensator zum Speichern einer elektrischen Ladung, ersten und zweiten Elektroden zum Übertragen eines Elektroschocks aus dem Kondensator auf den Patienten, und einer Impedanz-Meßvorrichtung zum Bestimmen der Transthorax-Impedanz des Patienten durch periodisches Abtasten der Transthorax-Impedanz vorgesehen ist. Der niedrigste Wert der abgetasteten Transthorax-Impedanz wird bestimmt, und unabhängig davon wird ein zum Abgeben zu einem Patienten erwünschter Strom gewählt. Die Ladung des Kondensators wird unterbrochen, wenn die gespeicherte Ladung zum Abgeben des ausgewählten Stroms ausreichend ist. Dieser Strom wird im Betrieb unabhängig von der gemessenen Transthorax-Impedanz abgegeben.

Auch hier ist eine echte Verteilung der Stromdichte auf unterschiedliche Zonen des Herzmuskels kaum möglich, weil nur zwei Elektroden verwendet werden.

Bei einem weiteren, aus den US-A- 4 708 145 bekannten Defibrillator mit drei Elektroden erfolgt die Abgabe von Defibrillierungsimpulsen sequentiell zwischen einer ersten Elektrode und jeweils einer der beiden anderen Elektroden, wobei auch hier die Impulse durch ein Zeitintervall voneinander getrennt sind.

Gemäß dem Gegenstand der älteren europäischen Patentanmeldung mit der Anmeldenummer 92104392.3 (Veröffentlichungnummer EP-A-0 505 857) wird eine räumlich optimale Stromverteilung dadurch erreicht, daß die einzelnen Elektroden an ihnen jeweils zugeordneten Ausgangsanschlüssen von mehreren in Reihe geschalteten Ausgängen einer Einrichtung angeschlossen ist, die an jedem ihrer Ausgänge jeweils gleichzeitig einen elektrischen Impuls abgibt.

Der Erfindung liegt die Aufgabe zugrunde, einen Defibrillator bzw. Kardiovertierer anzugeben, mit dem sich eine sowohl räumlich als auch zeitlich optimale Stromverteilung im Herzen zur Erzielung einer möglichst effektiven Defibrillation einstellen läßt.

Gemäß der Erfindung wird die Aufgabe dadurch gelöst, daß bei dem Defibrillator/Kardiovertierer der eingangs angegebenen Art die Einrichtung zur Erzeugung der elektrischen Impulse n-1 in Reihe geschaltete Ausgänge aufweist, an deren insgesamt n Ausgangsanschlüssen die Elektroden angeschlossen sind, daß die impulserzeugende Einrichtung an jedem ihrer Ausgänge jeweils gleichzeitig einen elektrischen Impuls abgibt, daß an den Elektroden eine Meßeinrichtung zur Erfassung einer von der Anordnung der Elektroden in bezug auf das Herzgewebe abhängigen elektrischen Meßgröße angeschlossen ist und daß Mittel zum individuellen Einstellen der Impulshöhen der Impulse an den Ausgängen der impulserzeugenden Einrichtung in Abhängigkeit von der erfaßten Meßgröße vorgesehen sind. Dadurch wird erreicht daß bei der Defibrillation oder Kardiovertierung des Herzens die Stromverteilung über das Herzmuskelgewebe über die Impulshöhen, d.h. elektrischen Spannungen an den Ausgängen der impulserzeugenden Einrichtung einstellbar ist, wobei bei der Einstellung der Stromverteilung die jeweilige Anordnung der Elektroden in bezug auf das Herz und die geometrische Verteilung des Herzgewebes zwischen den Elektroden berücksichtigt wird.

Entsprechend einer bevorzugten Ausbildung des erfindungsgemäßen Defibrillators/Kadiovertierers besteht die Meßeinrichtung aus einer Impedanzmeßeinrichtung zur Messung der elektrischen Impedanz zwischen den Elektroden. Der damit erhaltene Impedanzmeßwert gibt eine zuverlässige Information über die räumliche Verteilung der Herzmuskelmasse in bezug auf die Anordnung der Elektroden, so daß darüber eine optimale Einstellung der Impulshöhen der Impulse zur Erzielung einer vorgegebenen Defibrillierungsstromdichte im Herzmuskel möglich ist.

Bei einer alternativen Ausbildung der Meßeinrichtung besteht diese zumindest aus einer zwischen einem der Ausgangsanschlüsse der impulserzeugenden Einrichtung und der mit ihm verbundenen Elektrode angeordneten Strommeßeinrichtung, wobei die impulserzeugende Einrichtung zur Erfassung der Meßgröße an zumindest einem ihrer Ausgänge einen Meßimpuls erzeugt. Dabei kann aus dem von dem Meßimpuls erzeugten Stromfluß auf die räumliche Verteilung der Herzmuskelmasse zwischen den Elektroden geschlossen werden.

Um insbesondere bei einer Ausbildung des erfindungsgemäßen Defibrillator/Kardiovertierer als implantierbares Gerät die Impulshöhen an den Ausgängen der impulserzeugenden Einrichtung für eine gewünschte Stromverteilung im Herzen vorprogrammieren zu können, ist in vorteilhafter Weise vorgesehen, daß die Mittel zum Einstellen der Impulshöhen mit einem Parameterspeicher verbunden sind, in dem Parameterwerte für die Impulshöhen abgespeichert sind, und daß der Parameterspeicher mit einer Telemetrieeinrichtung zur Übertragung der Parameterwerte zwischen dieser und einem Programmiergerät verbunden ist. Dabei können die von der Meßeinrichtung gelieferten Meßwerte beispielsweise auf einer Anzeigeeinrichtung des Programmiergeräts zur Anzeige gebracht werden und der Bedienperson Hinweise zur Programmierung der einzelnen Paramterwerte für die Impulshöhen geben, um die gewünschte Stromverteilung im Herzen zu erreichen.

Entsprechend einer vorteilhaften Ausbildung der erfindungsgemäßen Defibrillators/Kardiovertierers weist die impulserzeugende Einrichtung n-1 in Reihe geschaltete Kondensatoren auf, die zum Aufladen auf unterschiedliche vorgegebene Ladespannungen über eine Schalteranordnung an eine Ladeschaltung schaltbar sind; ferner sind die Anschlußpunkte der einzelnen Kondensatoren über steuerbare Schalter mit den Ausgangsanschlüssen der impulserzeugenden Einrichtung verbunden. Solange die Schalteranordnung geschlossen ist, werden die Kondensatoren auf die jeweils vorgesehenen Ladespannungen aufgeladen, wobei entweder die Kondensatoren gleichzeitig mit ihnen jeweils zugeordneten Spannungsausgängen der Ladeschaltung verbunden werden oder einzeln nacheinander an einen einzigen Spannungsausgang der Ladeschaltung geschaltet werden. Die Impulsabgabe an die Elektroden erfolgt danach durch gleichzeitiges Schließen der steuerbaren Schalter.

Um die Stromverläufe über die einzelnen Elektroden zusätzlich beeinflussen können, sind in vorteilhafter Weise in den Leitungswegen zwischen den Ausgangsanschlüssen der impulserzeugenden Einrichtung und den Elektroden passive elektrische Bauelemente, vorzugsweise Induktivitäten und/oder Widerstände eingefügt. Damit wird für den Fall eines Kurzschlusses zwischen den Elektroden eine Strombegrenzung erreicht. Außerdem können Stromverläufe erzielt werden, die von einem exponentiell abklingeden Verlauf, wie er bei einer einfachen Kondensatorentladung auftritt, abweichen und beispielsweise die Form einer stark gedämpften Sinusschwingung aufweisen; eine derartige Impulsform hat sich für Debibrillierungszwecke als besonderrs wirksam erwiesen.

Zur weiteren Erläuterung der Erfindung wird im folgenden auf die Figuren der Zeichnung Bezug genommen; im einzelnen zeigen:
- FIG. 1: ein erstes Ausführungsbeispiel des erfindungsgemäßen Defibrillators/Kardiovertierers mit einer Impedanzmeßeinrichtung,
- FIG. 2: ein weiteres Ausführungsbeispiel mit einer Strommeßeinrichtung und
- FIG. 3: ein Ausführungsbeispiel für die impulserzeugende Einrichtung, wie sie in FIG 1 und 2 dargestellt ist.

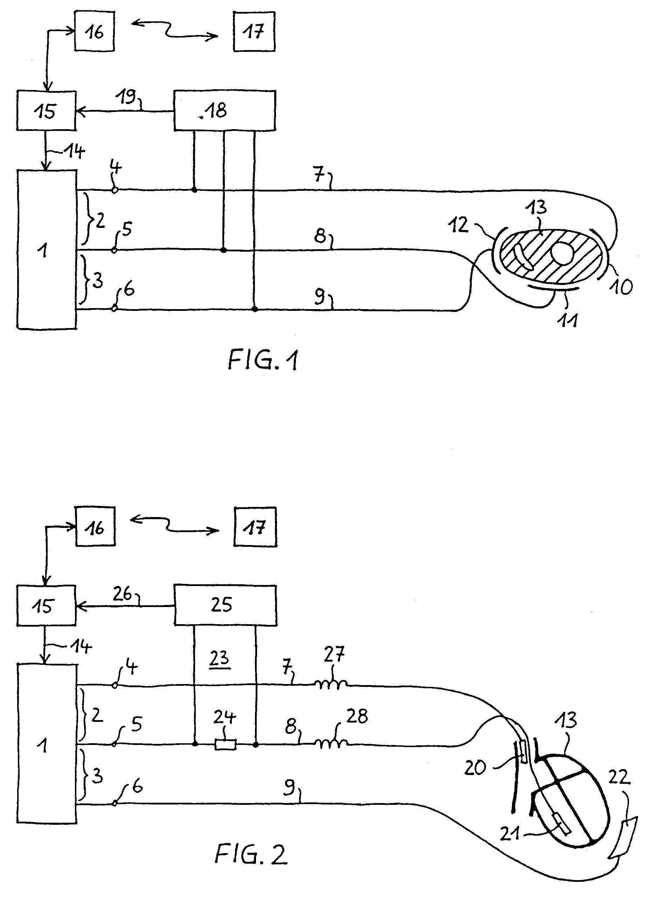

Bei dem in Figur 1 gezeigten Blockschaltbild eines implantierbaren Defibrillators oder Kardiovertierers ist mit 1 eine Einrichtung zur Erzeugung elektrischer Impulse bezeichnet. Die impulserzeugende Einrichtung 1 weist zwei in Reihe geschaltete Ausgänge 2 und 3 auf, deren insgesamt drei Ausgangsanschlüssen 4,5 und 6 über Elektrodenzuleitungen 7,8 und 9 mit drei Elektroden 10,11 und 12 verbunden sind, welche an einem hier im Querschnitt dargestellten Herzen 13 angeordnet sind. Die gezeigte Anordnung der Elektroden 10,11 und 12 in bezug auf das Herz 13 ist nur als eines von vielen Beispielen zu verstehen. Beispielsweise können die Elektroden 10,11 und 12 auch in der vena cava, in dem rechten Ventrikel sowie subkutan, dem linken Ventrikel des Herzens gegenüberliegend angeordnet sein.

Die impulserzeugende Einrichtung 1 ist über eine Steuerleitung 14 mit einem Parameterspeicher 15 verbunden, in dem unterschiedliche Werte für die Impulshöhen der an den Ausgängen 1 und 3 zu erzeugenden Impulse abgespeichert sind. Diese Werte können mittels einer an dem Parameterspeicher 15 angeschlossenen Telemetrieeinrichtung 16 zwischen dem Parameterspeicher 15 und einem externen Programmiergerät 17 übertragen werden. An den Ausgangsanschlüssen 4,5 und 6 ist eine Impedanzmeßeinrichtung 18 angeschlossen, die zu vorgegebenen Zeitpunkten die elektrische Impedanz des Herzgewebes 13 zwischen den Elektroden 10,11 und 12 mißt. In der Impedanzmeßeinrichtung 18 wird die gemessene Impedanz in der Weise ausgewertet, daß eine Information über die räumliche Anordnung der Elektrode 10,11 und 12 in bezug auf das Herz 13 und über die Verteilung der Herzmuskelmasse 13 zwischen den Elektroden 10,11 und 12 erhalten wird. Wie nämlich Figur 1 zeigt, ist die Herzmuskelmasse sehr ungleichmäßig über den Querschnitt des Herzens 13 verteilt. Außerdem können die Elektroden 10,11 und 12, wie oben bereits erläutert an sehr unterschiedlichen Positionen in bezug auf das Herz 13 angeordnet werden. Durch Impedanzmessung zwischen den drei Elektroden 10,11 und 12 können diese Verhältnisse meßtechnisch auf einfache Weise erfaßt werden. Das so ausgewertete Impedanmeßsignal wird über eine Steuerleitung 19 dem Parameterspeicher 15 zugeführt. In dem Parameterspeicher 15 erfolgt entweder eine automatische Anpassung der programmierten Werte für die Impulshöhen der zu erzeugenden Impulse an das gemessene Impedanzmeßsignal oder das Impedanzmeßsignal wird über die Telemetrieeinrichtung 15 an das Programmiergerät 17 übertragen und dort zur Anzeige gebracht, um der Bedienperson zusätzliche Informationen für die Programmierung der Impulshöhen zu geben. Darüberhinaus können durch die Bedienperson Randbedingungen programmiert werden, unter denen die automatische Impulshöheneinstellung durch die Meßeinrichtung 18 erfolgt.

Zur Defibrillation des Herzens 13 werden an den Ausgängen 2 und 3 der impulserzeugenden Einrichtung 1 gleichzeitig Defibrillierungsimpulse erzeugt, deren jeweilige Impulshöhe so eingestellt ist, daß im Herzen 13 eine gewünschte Stromverteilung erreicht wird. Dabei ist der Einfluß der Herzgeometrie und der Anordnung der Elektroden 10,11 und 12 auf die Stromverteilung durch die vorangegangene Impdanzmessung und entsprechende Anpassung der Impulshöhen im voraus bei der Einstellung der Impulshöhen berücksichtigt.

Figur 2 zeigt ein weiteres Ausführungsbeispiel für den erfindungsgemäßen Defibrillator/Kardiovertierer, bei dem ebenso, wie die Figur 1 gezeigt, eine impulserzeugende Einrichtung 1 an ihren zwei in Reihe geschalteten Ausgängen 2 und 3 mit den insgesamt drei Ausgangsanschlüssen 4,5 und 6 über Elektrodenzuleitungen 7,8 und 9 mit im Bereich des Herzens 13 angeordneten Elektroden 20,21 und 22 verbunden ist. Zwischen dem Ausgangsanschluß 5 und der mit ihm verbundenen Elektrode 21 ist eine Strommeßeinrichtung 23 bestehend aus einem Meßwiderstand 24 und einer parallel dazu angeordneten Spannungsmeßeinrichtung 25 angeordnet. Die Strommeßeinrichtung 23 bzw. Spannungsmeßeinrichtung 25 ist ausgangsseitig über eine Steuerleitung 26 mit einem Parameterspeicher 15 verbunden, in dem Parameterwerte zur Einstellung der Impulshöhen an den Ausgängen 2 und 3 der impulserzeugenden Einrichtung 1 enthalten sind. Der Parameterspeicher 15 ist dazu über eine Steuerleitung 14 an der impulserzeugenden Einrichtung 1 angeschlossen und kommuniziert darüberhinaus über eine Telemetrieeinrichtung 16 mit einem Programmiergerät 17. Schließlich sind im Verlauf der Elektrodenzuleitungen 7 und 8 zwei Induktivitäten 27 und 28 angeordnet, die zur Stromimpulsformung und Kurzschlußstrombegrenzung dienen.

Um eine Information über die Anordnung der Elektroden 20, 21 und 22 und die räumliche Verteilung der Herzmuskelmasse zwischen den Elektroden 20,21 und 22 zu erhalten, erzeugt die impulserzeugende Einrichtung 1 an ihren Ausgängen 2 und 3 jeweils gleichzeitig Meßimpulse mit definierter, jedoch weit unter der Defibrillierungsschwelle des Herzens 13 liegender Impulshöhe. Der daraus resultierende Stromverlauf in der Elektrodenzuleitung 8 ist von der Elektrodenkonfiguration in bezug auf das Herz 13 abhängig und wird, wie für das Ausführungsbeispiel nach Figur 1 beschrieben, zur Einstellung bzw. Korrektur der Impulshöhe der an den Ausgängen 2 und 3 zu erzeugenden Impulse herangezogen.

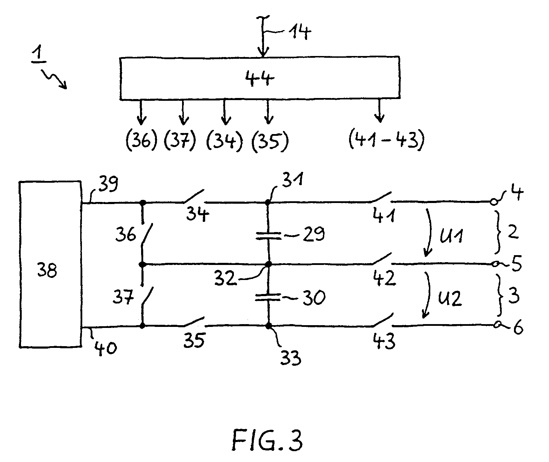

Figur 3 zeigt ein Ausführungsbeispiel für die impulserzeugende Einrichtung 1 mit zwei in Reihe geschalteten Kondensatoren 29 und 30 die an ihren Anschlußpunkten 31,32 und 33 über eine steuerbare Schalteranordnung bestehend aus vier einzeln steuerbaren Schaltern 34,35,36 und 37 an einer Ladeschaltung 38 angeschlossen sind. Dabei verbinden die beiden Schalter 34 und 35 die jeweils äußeren Anschlußpunkte 31 und 33 der Reihenschaltung der Kondensatoren 29 und 30 mit den beiden Ausgangsanschlüssen 39 und 40 des Spannungsausgangs der Ladeschaltung 38, während die Schalter 36 und 37 den mittleren Anschlußpunkt 32 mit den beiden Ausgangsanschlüssen 39 und 40 verbinden. Die Anschlußpunkte 31,32 und 33 der Kondensator 29 und 30 sind ferner über drei jeweils gleichzeitig steuerbare Schalter 41,42 und 43 mit den Ausgangsanschlüssen 4,5 und 6 der impulserzeugenden Einrichtung 1 verbunden. Den steuerbaren Schaltern 34 bis 37 und 41 bis 43 ist eine sie steuernde Steuereinrichtung 44 zugeordnet, die ihrerseits über die Steuerleitung 14 von dem Parameterspeicher 15 (Figuren 1,2) gesteuert wird.

Um den Kondensator 29 auf eine erste Spannung U1 und den Kondensator 30 auf eine zweite Spannung U2 aufzuladen, werden zunächst die Schalter 34 und 37 solange geschlossen, bis der Kondensator 29 auf die Spannung U1 aufgeladen ist. Anschließend werden bei geöffneten Schaltern 34 und 37 die Schalter 35 und 36 geschlossen; erreicht die Spannung an dem Kondensator 30 den Wert U2, so werden die Schalter 35 und 36 geöffnet. Zur Impulsabgabe mit der Spannung U1 am Ausgang 2 und der Spannung U2 am Ausgang 3 werden die Schalter 41,42 und 43 geschlossen.

### Bezugszeichenliste

- 1: impulserzeugende Einrichtung
- 2,3: Ausgänge von 1
- 4,5,6: Ausgangsanschlüsse von 2,3
- 7,8,9: Elektrodenzuleitungen
- 10,11,12: Elektroden
- 13: Herz
- 14: Steuerleitung
- 15: Parameterspeicher
- 16: Telemetrieeinrichtung
- 17: Programmiergerät
- 18: Impedanzmeßeinrichtung
- 19: Steuerleitung
- 20,21,22: Elektroden
- 23: Strommeßeinrichtung
- 24: Strommeßwiderstand
- 25: Spannungsmeßeinrichtung
- 26: Steuerleitung
- 27,28: passive elektische Bauelemente (Induktivitäten)
- 29,30: Kondensatoren
- 31,32,33: Anschlußpunkte von 29,30
- 34,35,36,37: Schalter einer steuerbaren Schalteranordnung
- 38: Ladeschaltung
- 39,40: Ausgangsanschlüsse von 38
- 41,42,43: steuerbare Schalter
- 44: Steuereinrichtung für 34-37,41-43

## Patentansprüche

1. Defibrillator/Kardiovertierer system mit einer Mehrzahl von n, n≥3, im Bereich des Herzens (13) anordnungsbaren Elektroden (10,11,12), die an einer Einrichtung (1) zur Erzeugung elektrischer Impulse angeschlossen sind, wobei die Einrichtung (1) zur Erzeuggung der elektrischen Impulse n-1 in Reihe geschaltete Ausgänge (2,3) aufweist, deren insgesamt n Ausgangsanschüssen (4,5,6) mit den Elektroden (10,11,12;20,21,22) über Elektrodenzuleitungen (7,8,9) sind, dadurch gekennzeichnet daß die impulserzeugende Einrichtung (1) an jedem ihrer Ausgänge (2,3) jeweils gleichzeitig einen elektrischen Impuls abgibt, daß an den Elektroden (10,11,12;20,21,22) eine Meßeinrichtung (18;23) zur Erfassung einer von der Anordnung der Elektroden (10,11,12;20,21,22) in bezug auf das dazwischenliegende Herzgewebe (13) abhängigen elektrischen Meßgröße angeschlossen ist und daß Mittel zum individuellen Einstellen der Impulshöhen der Impulse an den Ausgängen (2,3) der impulserzeugenden Einrichtung (1) in Abhängigkeit von der erfaßten Meßgröße vorgesehen sind.

2. Defibrillator Kardiovertierer nach Anspruch 1, **dadurch gekennzeichnet**, daß die Meßeinrichtung aus einer Impedanzmeßeinrichtung (18) zur Messung der elektrischen Impedanz zwischen den Elektroden (10,11,12) besteht.

3. Defibrillator Kardiovertierer nach Anspruch 1, **dadurch gekennzeichnet**, daß die Meßeinrichtung zumindest aus einer zwischen einem der Ausgangsanschlüsse (z.B.5) der impulserzeugenden Einrichtung (1) und der mit ihm verbundenen Elektrode (21) angeordneten Stromeinrichtung (23) besteht und daS die impulserzeugende Einrichtung (1) zur Erfassung der Meßgröße an zumindest einem ihrer Ausgänge (2,3) einen Meßimpuls erzeugt.

4. Defibrillator/Kardiovertierer nach einem der vorangehenden Ansprüche, **dadurch gekennzeichnet**, daß die Mittel zum Einstellen der Impulshöhen mit einem Parameterspeicher (15) verbunden sind, in dem Parameterwerte für die Impulshöhen abgespeichert sind, und daß der Parameterspeicher (15) mit einer Telemetrieeinrichtung (16) zur Übertragung der Parameterwerte zwischen dieser und einem Programmiergerät (17) verbunden ist.

5. Defibrillator/Kardiovertierer nach einem der vorangehenden Ansprüche, **dadurch gekennzeichnet**, daß die impulserzeugende Einrichtung (1) n-1 in Reihe geschaltete Kondensatoren (29,39) aufweist, die zum Aufladen auf unterschiedliche vorgegebene Ladespannungen (U1,U2) über eine Schalteranordnung (34-37) an eine Ladeschaltung (38) schaltbar sind, und daß die Anschlußpunkte (31,32,33) der einzelnen Kondensatoren (29,30) über steuerbare Schalter (41,42,43) mit den Ausgangsanschlüssen (4,5,6) der impulserzeugenden Einrichtung (1) verbunden sind.

6. Defibrillator/Kardiovertierer nach einen der vorangehenden Ansprüche, **dadurch gekennzeichnet**, daß in den Leitungswegen zwischen den Ausgangsanschlüssen (4,5,6) der impulserzeugenden Einrichtung (1) und den mit ihnen verbundenen Elektroden (20,21,22) passive elektrische Bauelemente (27,28) eingefügt sind.

## Claims

1. Defibrillator/cardioverter (system) having a plurality of n, n ≥ 3, electrodes (10, 11, 12), which can be arranged in the region of the heart (13) and are connected to a device (1) for generating electrical pulses, with the device (1) for generating the electrical pulses having n-1 series-connected outputs (2, 3), the total of n output terminals (4, 5, 6) of which are connected to the electrodes (10, 11, 12; 20, 21, 22) by way of electrode supply lines (7, 8, 9), characterised in that the pulse-generating device (1) gives out an electrical pulse at each of its outputs (2, 3), simultaneously in each case, in that there is connected to the electrodes (10, 11, 12; 20, 21, 22) a measuring device (18, 23) for detecting an electrical measured variable which is dependent on the arrangement of the electrodes (10, 11, 12; 20, 21, 22) with respect to the heart tissue (13) between them, and in that there are provided means for the individual adjustment of the pulse heights of the pulses at the outputs (2, 3) of the pulse-generating device (1) as a function of the measured variable which is detected.

2. Defibrillator/cardioverter according to claim 1, characterised in that the measuring device comprises an impedance measuring device (18) for measuring the electrical impedance between the electrodes (10, 11, 12).

3. Defibrillator/cardioverter according to claim 1, characterised in that the measuring device comprises at least one current device (23) arranged between one of the output terminals (e.g. 5) of the pulse-generating device (1) and the electrode (21) connected thereto, and in that for detecting the measured variable, the pulse-generating device (1) generates a measuring pulse at at least one of its outputs (2, 3).

4. Defibrillator/cardioverter according to one of the preceding claims, characterised in that the means for adjusting the pulse heights are connected to a parameter memory (15), in which parameter values for the pulse heights are stored, and in that the parameter memory (15) is connected to a telemetry device (16) for transmitting the parameter values between the said parameter memory and a programming unit (17).

5. Defibrillator/cardioverter according to one of the preceding claims, characterised in that the pulse-generating device (1) has n-1 series-connected capacitors (29, 39 (sic)), which, for charging to different specified charging voltages (U1, U2), can be connected by way of a switch arrangement (34-37) to a charging circuit (38), and in that the terminals (31, 32, 33) of the individual capacitors (29, 30) are connected by way of controllable switches (41, 42, 43) to the output terminals (4, 5, 6) of the pulse-generating device (1).

6. Defibrillator/cardioverter according to one of the preceding claims, characterised in that inserted into the line paths between the output terminals (4, 5, 6) of the pulse-generating device (1) and the electrodes (20, 21, 22) connected thereto are passive electrical components (27, 28).

## Revendications

1. Système défibrillateur/cardioverteur comportant une pluralité de n, n ≥ 3, électrodes (10, 11, 12) qui peuvent être disposées dans la zone du coeur (13) et qui sont raccordées à un dispositif (1) de production d'impulsions électriques, le dispositif (1) de production des impulsions électriques comportant n-1 sorties (2, 3) montées en série, dont les en tout n bornes (4, 5, 6) de sortie sont raccordées aux électrodes (10, 11, 12;20,21,22) par l'intermédiaire de lignes (7, 8, 9) d'alimentation des électrodes, caractérisé en ce que le dispositif (1) qui produit des impulsions fournit à chacune de ses sorties (2, 3) en même temps une impulsion électrique, en ce qu'il est raccordé aux électrodes (10, 11, 12; 20, 21, 22) un dispositif (18;23) de mesure pour détecter une grandeur de mesure électrique dépendant de la disposition des électrodes (10, 11, 12; 20, 21, 22) par rapport à la paroi (13) du coeur se trouvant au milieu et en ce qu'il est prévu des moyens pour régler individuellement le niveau des impulsions aux sorties (2, 3) du dispositif (1) de production d'impulsions en fonction de la grandeur de mesure détectée.

2. Défibrillateur/cardioverteur suivant la revendication 1, caractérisé en ce que le dispositif de mesure est constitué d'un dispositif (18) de mesure d'impédance pour mesurer l'impédance électrique entre les électrodes (10, 11, 12).

3. Défibrillateur/cardioverteur suivant la revendication 1, caractérisé en ce que le dispositif de mesure est constitué d'au moins un dispositif (23) d'alimentation en courant monté entre l'une des bornes (par exemple 5) de sortie du dispositif (1) producteur d'impulsions et l'électrode (21) qui lui est reliée et en ce que le dispositif (1) producteur d'impulsions produit une impulsion de mesure à au moins l'une de ses sorties (2, 3) pour détecter la grandeur de mesure.

4. Défibrillateur/cardioverteur suivant l'une des revendications précédentes, caractérisé en ce que les moyens pour régler les niveaux d'impulsion sont reliés à une mémoire (15) de paramètres, dans laquelle des valeurs de paramètres pour les niveaux d'impulsion sont enregistrées, et en ce que la mémoire (15) de paramètres est reliée à un dispositif (16) de télémétrie pour transmettre les valeurs de paramètres entre celui-ci et un appareil (17) de programmation.

5. Défibrillateur/cardioverteur suivant l'une des revendications précédentes, caractérisé en ce que le dispositif (1) producteur d'impulsions comporte n-1 condensateurs (29,39), condensateurs branchés en série, qui, pour charger des tensions (U1, U2) de charge prescrites différentes, peuvent être branchées à un circuit (38) de charge par l'intermédiaire d'un dispositif (34 à 37) de commutation et en ce que les points (31, 32, 33) de raccordement des condensateurs (29, 30) individuels sont reliés, par l'intermédiaire d'interrupteurs (41, 42, 43) pouvant être commandés, aux bornes (4, 5, 6) de sortie du dispositif (1) producteur d'impulsions.

6. Défibrillateur/cardioverteur suivant l'une des revendications précédentes, caractérisé en ce qu'il est inséré des composants (27, 28) électriques passifs dans les voies de ligne entre les bornes (4, 5, 6) de sortie du dispositif (1) producteur d'impulsions et les électrodes (20, 21 22) qui y sont reliées.
